# EUROPÄISCHE PATENTSCHRIFT

(11) **EP 0 844 240 B1**
(45) Veröffentlichungstag und Bekanntmachung des Hinweises auf die Patenterteilung: **14.02.2001**
(21) Anmeldenummer: 97120566.1
(22) Anmeldetag: 24.11.1997
(51) Int. Cl.: C07D 201/16

(54) **Verfahren zur Rückgewinnung von Caprolactam und seinen Oligomeren aus wässrigen Polycaprolactam-Extrakten**
Process for recovering caprolactam and its oligomers form aqueous polycaprolactam extracts
Procédé de récupération de caprolactame et ses oligomères à partir d'extraits de polycaprolactame aqueux

(30) Priorität: 25.11.1996 DE 19648747
(43) Veröffentlichungstag der Anmeldung: 27.05.1998
(73) Patentinhaber: BASF AKTIENGESELLSCHAFT, 67056 Ludwigshafen (DE)
(72) Erfinder: Egly, Horst, 67459 Böhl-Iggelheim (DE); Hünger, Hans-Harald, 67158 Ellerstadt (DE); Sauer, Thomas, Dr., 67246 Dirmstein (DE)
(74) Vertreter: Kinzebach, Werner, Dr.

(56) Entgegenhaltungen:
- EP-A- 0 065 168
- EP-A- 0 137 124
- EP-A- 0 279 439
- EP-A- 0 633 246
- WO-A-88/04651
- WO-A-97/34868
- DE-A- 1 944 910
- DE-A- 4 129 076
- US-A- 4 311 642
- US-A- 5 294 707

## Beschreibung

Die vorliegende Erfindung betrifft ein Verfahren zur Rückgewinnung von Caprolactam und seinen Oligomeren durch Entfernung anorganischer Verunreinigungen aus wässrigen Polycaprolactam-Extrakten.

Polycaprolactam ist das technisch wichtigste Polyamid. An seine Qualität werden hohe Anforderungen gestellt. Polycaprolactam enthält herstellungsbedingt in der Regel noch 10 bis 15 Gew.-% niedermolekulare Anteile, die sich bei der Weiterverarbeitung oft störend auswirken oder die Qualität der Fertigerzeugnisse ungünstig beeinflussen. So wirkt monomeres Caprolactam und seine niedermolekularen Oligomere deutlich weichmachend auf das Polymerisat. Diese niedermolekularen Anteile können durch wässrige Extraktion aus dem Polycaprolactam entfernt werden (vgl. v. Hopf, Müller, Wenger in "Die Polyamide", S. 26 sowie DRP 766120).

Die so gewonnenen Lösungen von Caprolactam und seinen niedermolekularen Oligomeren enthalten in der Regel noch anorganische Verunreinigungen. So weisen beispielsweise Extrakte von pigmentiertem Polycaprolactam neben den Pigmenten selber noch meist Oxide des Siliciums oder des Aluminiums aus der Coatingschicht der Pigmente auf, die nicht durch Filtration abgetrennt werden können. Diese anorganischen Verunreinigungen müssen vor der Rückführung des Caprolactams bzw. seiner Oligomere in den Polymerisationsprozess entfernt werden.

Die Rückgewinnung des Caprolactams aus diesen Extrakten erfolgt in der Regel durch Entfernen des Wassers in üblichen Verdampferanlagen und anschliessende Destillation des Caprolactams. Hierbei verbleiben die schwerflüchtigen Oligomere sowie die anorganischen Verunreinigungen im Destillationsrückstand. Dieser kann zur Rückgewinnung des restlichen Caprolactams einer Oligomerenspaltung unterworfen werden. Als Nachteil dieses Rückgewinnungsverfahrens erweist sich, daß die für eine Depolymerisation notwendigen Reaktionsbedingungen sich für die Spaltöfen in hohem Maße als abrasiv und korrosiv erweisen. Zudem wirken die anorganischen Bestandteile als Katalysatorgift, so daß nach einiger Zeit die Depolymerisation nicht mehr selektiv abläuft. Die Spaltöfen weisen daher nur kurze Betriebsintervalle auf, bevor sie wieder gereinigt werden müssen.

Die US 4,311,642 beschreibt ein Verfahren zur Rückgewinnung von Caprolactam aus aufkonzentrierten Extrakten der Nylon-6-Herstellung, bei dem man das wässrige, Caprolactam enthaltende Extrakt in einen Dünnschichtverdampfer bei einer Arbeitstemperatur von 200°C bis 300°C und einem Druck von 10 bis 250 Torr gibt. Wasser und ein Teil des Caprolactams werden am Verdampferkopf abgeführt; schwerflüchtige Caprolactamoligomere und weiteres Caprolactam fallen als Sumpfprodukt an, welches einer Depolymerisation zugeführt wird.

Der vorliegenden Erfindung liegt somit die Aufgabe zugrunde, ein neues Verfahren bereitzustellen, das es erlaubt, Caprolactam und seine Oligomere frei von anorganischen Verunreinigungen aus wässrigen Polycaprolactamextrakten zurückzugewinnen, ohne daß ein Depolymerisationsschritt notwendig ist.

Die Aufgabe konnte gelöst werden durch ein Verfahren zur Rückgewinnung von Caprolactam und seinen Oligomeren durch Entfernung anorganischer Verunreinigungen aus wässrigen Polycaprolactam-Extrakten, wobei man die wässrigen Extrakte kontinuierlich durch einen Verdampfer führt, dadurch gekennzeichnet, daß das Verhältnis von Flüssigkeitsmenge am Verdampferrohraustritt zum Rohrumfang unterhalb 200 l/(m·h) liegt, wobei es zu einer zumindest partiellen Entnetzung auf der Verdampferoberfläche kommt und sich die anorganischen Verunreinigungen auf den Wärmetauscheroberflächen des Verdampfers abscheiden.

Erfindungsgemäß wird der wässrige Polycaprolactam-Extrakt kontinuierlich in dünnen Schichten über die Wärmetauscheroberflächen des Verdampfers geführt, wobei die Temperatur der Wärmetauscheroberflächen oberhalb der Siedetemperatur des wässrigen Extrakts liegt. Hierbei wird das Verhältnis von Flüssigkeitsmenge zu Verdampferrohrgeometrie so bemessen, daß es zur zumindest partiellen Entnetzung auf der Verdampferoberfläche kommt. Dies ist dann gewährleistet, wenn das Verhältnis der am Verdampferaustritt ablaufenden Flüssigkeitsmenge zum Rohrumfang des Verdampfers unterhalb 200 l/(m·h), vorzugsweise im Bereich von 30 bis 100 l/(m·h) liegt.

In einer besonders bevorzugten Ausführungsform des erfindungsgemäßen Verfahrens werden Temperatur und Flüssigkeitsmenge so bemessen, daß der Verdampferaustrag aus einer nahezu wasserfreien Caprolactam/Oligomer-Schmelze besteht. Hierzu sind vorzugsweise Temperaturen an den Wärmeaustauscheroberflächen des Verdampfers oberhalb 220°C, insbesondere oberhalb 235°C erforderlich. Vorzugsweise arbeitet man bei Normaldruck oder einem leichten Überdruck. Das erfindungsgemäße Verfahren kann jedoch auch bei Unterdruck durchgeführt werden.

Für das erfindungsgemäße Verfahren sind im Prinzip alle Verdampfungstechniken geeignet, bei denen die einzudampfende Flüssigkeit in dünnen Filmen über die Wärmetauscheroberflächen geführt werden. Hierzu zählen Plattenfilmverdampfer, Fallstrom- oder Fallfilmverdampfer (s.a. Ullmanns Enzyklopädie der technischen Chemie, 4. Aufl., Bd. 2, S. 656 f.). Bevorzugt werden jedoch handelsübliche Fallfilmverdampfer verwendet. Die Beheizung erfolgt nach verfahrenstechnisch üblichen Methoden mit Wasserdampf oder anderen Wärmeträgermedien.

Die Hauptmenge an Caprolactam bzw. seinen Oligomeren wird im Verdampferaustrag in Form einer hochkonzentrierten Lösung oder einer Schmelze, vorzugsweise mit einem Wassergehalt < 7 % und besonders < 2 %, bezogen auf den Austrag, gewonnen. Zur Rückführung des bei der Verdampfung des Wassers mitgeschleppten Caprolactams können die Brüden anschliessend nach üblichen Techniken, beispielsweise mittels Kolonnen, fraktioniert werden. Zur Vermeidung von Oxidationseffekten werden vorzugsweise alle Zuleitungen und Ableitungen sowie Vorrats- und Sammelbehältnisse mit Inertgas, vorzugsweise Stickstoff gespült.

Die Abreinigung der Wärmetauscheroberflächen von den abgelagerten anorganischen Bestandteilen erfolgt nach den üblichen Methoden, beispielsweise durch Abspritzen mit einem scharfen Wasserstrahl oder durch Abbürsten.

Das erfindungsgemäße Verfahren ist geeignet, übliche Polycaprolactam-Extrakte, wie sie bei dessen Herstellung anfallen, von anorganischen Verunreinigungen zu befreien und das darin enthaltene Caprolactam bzw. seine Oligomere zurückzugewinnen. Erfindungsgemäß bevorzugt sind Extrakte mit Caprolactamgehalten oberhalb 60 Gew.-%, insbesondere solche mit etwa 70 bis 75 Gew.-%. Derartige Lösungen werden in der Regel durch Aufkonzentrieren der wässrigen Polycaprolactam-Extrakte nach üblichen Verdampfungstechniken erhalten.

Das durch das erfindungsgemäße Verfahren zurückgewonnene Caprolactam bzw. seine Oligomere weisen eine Reinheit auf, die es erlaubt, sie in den Polymerisationsprozeß zurückzuführen.

Das im folgenden angegebene Ausführungsbeispiel soll die Erfindung erläutern, ohne sie jedoch einzuschränken.

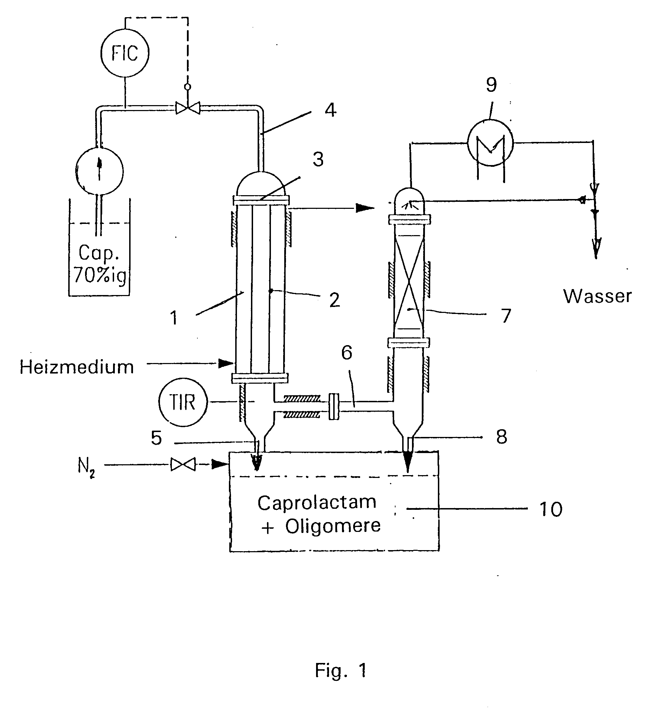

### Ausführungsbeispiel (Fig. 1)

Der Fallfilmverdampfer 1 ist mit Verdampferrohren 2 (Länge 1,5 m; Durchmesser 21 mm) ausgerüstet. Der Verdampfer wird mit einem flüssigen Wärmeträger beheizt. Auf den oberen Rohrboden 3 wird 3 bis 7 l 70 gew.-%iger, wässriger Polycaprolactam-Extrakt über die Zuleitung 4 gegeben. Die Temperatur im Verdampfer wird so eingeregelt, daß das im Austrag 5 befindliche Caprolactam eine Temperatur im Bereich von 235°C bis 250°C aufweist. Die Brüden werden über eine Dampfbrücke 6 in eine Kolonne 7 geführt. Hierbei trennt sich mitgeführtes Caprolactam ab, das über den Austrag 8 abgezowird. Der Wasserdampf wird über den Kolonnenkopf abgeführt und mittels eines Kondensators 9 kondensiert. Die Austräge 5 und 8 werden in das Vorratsgefäß 10 überführt, das zur Vermeidung von Oxidationseffekten über eine Stickstoffzuleitung mit Inertgas gespült wird.

## Patentansprüche

1. Verfahren zur Rückgewinnung von Caprolactam und seinen Oligomeren durch Entfernung anorganischer Verunreinigungen aus wässrigen Polycaprolactamextrakten, wobei man die wässrigen Extrakte kontinuierlich durch einen Verdampfer führt, dadurch gekennzeichnet, daß das Verhältnis von Flüssigkeitsmenge am Verdampferrohraustritt zum Rohrumfang unterhalb 200 l/(m·h) liegt, wobei es zu einer zumindest partiellen Entnetzung auf der Verdampferoberfläche kommt und sich die anorganischen Verunreinigungen auf den Wärmetauscheroberflächen des Verdampfers abscheiden.

2. Verfahren nach Anspruch 1, dadurch gekennzeichnet, daß es sich bei dem Verdampfer um einen Fallfilmverdampfer handelt.

3. Verfahren nach einem der vorhergehenden Ansprüche, dadurch gekennzeichnet, daß der wässrige Polycaprolactamextrakt wenigstens 60 Gew.-% Caprolactam und/oder dessen Oligomere enthält.

4. Verfahren nach einem der vorhergehenden Ansprüche, dadurch gekennzeichnet, daß die am Verdampferrohraustritt austretende Flüssigkeit eine hochkonzentrierte Caprolactam/Oligomer-Schmelze oder Lösung mit einem Wassergehalt < 7 % ist.

5. Verfahren nach einem der vorhergehenden Ansprüche, dadurch gekennzeichnet, daß das im Verdampferaustrag austretende Caprolactam und/oder dessen Oligomere eine Temperatur oberhalb 235°C aufweisen.

## Claims

1. A process for recovering caprolactam monomer and oligomer by removing inorganic contaminants from aqueous polycaprolactam extracts, where the aqueous extracts are passed continuously through an evaporator wherein the ratio of liquid volume at the evaporator tube exit to tube circumference is below 200 l/(m·h), where the evaporator surface is at least partially dewetted and the inorganic contaminants become deposited on the heat exchanger surfaces of the evaporator.

2. A process as claimed in claim 1, wherein the evaporator is a falling-film evaporator.

3. A process as claimed in any of the preceding claims, wherein the aqueous polycaprolactam extract comprises at least 60% by weight of caprolactam monomer and/or oligomer.

4. A process as claimed in any of the preceding claims, wherein the liquid leaving at the evaporator tube exit is a highly concentrated caprolactam/oligomer melt or solution with a water content < 7%.

5. A process as claimed in any of the preceding claims, wherein the caprolactam monomer and/or oligomer leaving in the evaporator effluent is at above 235°C.

## Revendications

1. Procédé de récupération de caprolactame et de ses oligomères par élimination d'impuretés inorganiques à partir d'extraits aqueux de polycaprolactame, dans lequel on conduit de manière continue les extraits aqueux à travers un évaporateur, caractérisé en ce que le rapport quantité de liquide à la sortie du tube d'évaporateur et périphérie du tube est inférieur à 200 l/(m·h), de façon à parvenir à une déréticulation au moins partielle à la surface de l'évaporateur et à une séparation des impuretés inorganiques sur les surfaces échangeuses de chaleur de l'évaporateur.

2. Procédé suivant la revendication 1, caractérisé en ce que, en ce qui concerne l'évaporateur, il s'agit d'un évaporateur à film descendant.

3. Procédé suivant l'une des revendications précédentes, caractérisé en ce que l'extrait aqueux de polycaprolactame contient au moins 60% en poids de caprolactame et/ou de ses oligomères.

4. Procédé suivant l'une des revendications précédentes, caractérisé en ce que le liquide sortant à la sortie du tube d'évaporateur est une masse fondue ou une solution ayant une teneur en eau inférieure à 7% de caprolactame/oligomère fortement concentrés.

5. Procédé suivant l'une des revendications précédentes, caractérisé en ce que le caprolactame et/ou ses oligomères sortant à la sortie de l'évaporateur présentent une température supérieure à 235°C.
